# EUROPEAN PATENT SPECIFICATION

(11) **EP 3 991 706 B1**
(45) Date of publication and mention of the grant of the patent: **15.05.2024**
(21) Application number: 20846533.6
(22) Date of filing: 15.07.2020
(51) Int. Cl.: A61F 13/15, A61F 13/49, A61F 13/496

(54) **METHOD FOR MANUFACTURING UNDERPANTS-TYPE WEARABLE ARTICLE**
VERFAHREN ZUR HERSTELLUNG VON UNTERHOSENARTIGEN TRAGBAREN ARTIKELN
PROCÉDÉ DE FABRICATION D'UN ARTICLE VESTIMENTAIRE DE TYPE SOUS-VÊTEMENT

(30) Priority: 26.07.2019 JP 2019137502
(43) Date of publication of application: 04.05.2022
(73) Proprietor: Zuiko Corporation, Settsu-shi Osaka 566-0045 (JP)
(72) Inventor: SHIMADA, Takahiro, Settsu-shi, Osaka 566-0045 (JP); FURUKAWA, Daisuke, Settsu-shi, Osaka 566-0045 (JP)
(74) Representative: Müller-Boré & Partner Patentanwälte PartG mbB
(86) International application number: PCT/JP2020/027428
(87) International publication number: WO 2021/020118

(56) References cited:
- WO-A1-2004/054490
- WO-A1-2018/154684
- CN-A- 107 205 855
- JP-A- 2008 136 943
- JP-A- 2013 081 658
- JP-A- 2014 117 860
- JP-A- 2016 112 166
- US-A1- 2012 202 664

## Description

### Technical Field

The present invention relates to a method for manufacturing an underpants-type wearable article having an elastic stretchable member.

### Background Art

Conventionally, various underpants-type wearable articles such as disposable underpants have been manufactured and sold. Such an underpants-type wearable article includes, as described in Patent Literature 1, a front side waist member and a back side waist member that are respectively disposed around front and back sides of a body, and an absorbing body disposed at a clotch portion. The front side waist member and the back side waist member are stretchable and shrinkable in a waist surrounding direction.

In the manufacture of the front side waist member and the back side waist member, first, between a pair of elongated nonwoven fabrics, a plurality of elastic stretchable members in a longitudinally elongated state are sandwiched. The pair of nonwoven fabrics are joined by heat fusing lines that intersect with a direction that the elastic stretchable members extend. As a result, a continuous body formed of the front side waist members and a continuous body formed of the back side waist members are formed respectively. With such a configuration, the elastic stretchable members are held between the pair of nonwoven fabrics.

After the continuous body formed of the front side waist members and the continuous body formed of the back side waist members are respectively formed, the absorbing bodies are disposed in a spaced-apart manner such that both end portions of absorbing body overlap with the front side waist member and the back side waist member respectively. Then, side fusing is applied to both side portions of the front side waist member and the back side waist member in a state where the front side waist member and the back side waist member overlap with each other. The respective continuous bodies of the front side waist members and the back side waist members are cut between wearable articles continuously disposed adjacent to each other, the manufacture of the wearable articles is completed.

In a case where the elastic stretchable member is not normally joined to the nonwoven fabric in the vicinity of a cutting portion of the wearable articles, that is, a joining defect occurs, the underpants-type wearable article cannot acquire a tension of an elastic stretchable member in the vicinity of side fusing portions at both end portions of a wearable article. Accordingly, there arises a drawback that wrinkles (pleats) having a predetermined shape cannot be obtained on the nonwoven fabric. In view of the above-mentioned circumstances, it is necessary to check a joining state of the elastic stretchable member with respect to the respective wearable articles.

In the above-mentioned manufacturing method, in a case where it is necessary to check whether or not the elastic stretchable member is normally joined to the nonwoven fabric in the vicinity of a cutting portion of the wearable article, such a check can be performed only after a cutting step where the continuous bodies of the waist members are cut. However, after the cutting step where the waist members are cut, wrinkles are generated in the nonwoven fabric due to a shrinkage of the elastic stretchable member. Accordingly, it is difficult to check a joining state of the elastic stretchable member.

Further, document JP 2016 112166 A describes a method for manufacturing a sheet-welded body, including a pre-irradiation holding process in which a belt-like sheet laminated body obtained by overlapping sheets is disposed on an outer surface of a supporting member having a light passage part that allows a laser beam to pass and is held in a pressurized state. Further including a laser irradiation process in which the belt-like laminated body is fused and cut by irradiating a planned region for forming a seal edge part of the sheet laminated body held on the supporting member in the pressurized state with the laser beam through the light passage part from the side of the supporting member, and simultaneously, a seal edge part is formed by welding cut edge parts, obtained by fusion cutting, of a plurality of sheets in the pressurized state. Further including a cutting means inserting process for subsequently inserting a cutting means in the planned region for forming the seal edge part irradiated with the laser beam in a thickness direction of the sheet laminated body.

Further, document US 2012/202664 A1 describes an apparatus and method to provide defect detection both before and after application of component patches to a traveling web to create a product. Web defect detection may be provided by way of at least one visual inspection station located upstream from the patch applicator. Patch defect detection may be accomplished by way of a visual inspection station located proximate the patch applicator. If defects are detected in either the traveling web or the component patch prior to patch application, patch application may be prevented until both a satisfactory web and patch are provided. If defects are detected after patch application, the resulting product may be culled.

### Citation List

### Patent Literature

Patent Literature 1: WO 2016/208513 A

### Summary of Invention

It is an object of the present invention to provide a method for manufacturing an underpants-type wearable article where a joining defect of an elastic stretchable member can be easily checked.

To solve the above problems, the method for manufacturing an underpants-type wearable article according to the present invention is a method for manufacturing an underpants-type wearable article that includes: a front side waist member disposed on a belly side of a body; a back side waist member disposed on a back side of the body; and an absorbing body disposed at a clotch portion of the body, both side portions of the front side waist member and the back side waist member being joined to each other by side joining using a pair of side joining portions, the method including: an elastic stretchable member sandwiching step of sandwiching a plurality of elastic stretchable members stretched in a predetermined stretching direction between a pair of sheets; a front side continuous body forming step of forming a continuous body of the front side waist members by intermittently joining, in the stretching direction, the pair of sheets and the elastic stretchable members in a stretched state; a back side continuous body forming step of forming a continuous body of the back side waist members by intermittently joining, in the stretching direction, the pair of sheets and the elastic stretchable member in a stretched state; a side joining step of intermittently joining the front side waist member and the back side waist member by the side joining portions in boundary regions which become boundaries of individual wearable articles in a state where the continuous body of the front side waist members and the continuous body of the back side waist members are overlapped with each other; an elastic stretchable member cutting step of cutting the elastic stretchable member of a continuous body of the front side waist members and the elastic stretchable members of the continuous body of the back side waist member in the boundary regions in the continuous body of the front side waist members and the continuous body of the back side waist members; a joining state inspection step of inspecting a joining state of the elastic stretchable members to the pair of sheet; and a continuous body cutting step of obtaining individual wearable articles by cutting the continuous body of the front side waist members and the continuous body of the back side waist members at the boundary regions.

### Brief Description of Drawings

FIG. 1 is a perspective view illustrating a basic configuration of an underpants-type wearable article manufactured by a manufacturing method of the present invention.
FIG. 2 is a developed view of the wearable article illustrated in FIG. 1.
FIG. 3 is a cross-sectional view taken along line III-III in FIG. 2.
FIG. 4 is a flowchart illustrating a series of steps of the manufacturing method as an embodiment of the manufacturing method of the underpants-type wearable article according to the present invention.
FIG. 5 is an explanatory plan view illustrating a rubber thread sandwiching step in the method for manufacturing an underpants-type wearable article according to the embodiment of the present invention.
FIG. 6 is an explanatory plan view illustrating a continuous body forming step in the method for manufacturing an underpants-type wearable article according to the embodiment of the present invention.
FIG. 7 is an explanatory plan view illustrating a side fusing step and a rubber thread cutting step in the method for manufacturing an underpants-type wearable article according to the embodiment of the present invention.
FIGS. 8A to 8C are explanatory plan views illustrating a joining state inspection step in the method for manufacturing an underpants-type wearable article according to the embodiment of the present invention, wherein FIG.8A is a view illustrating a state immediately after rubber threads are cut, FIGS.8B and 8C are views illustrating a manner how a joining defect of the rubber threads in the vicinity of a joining portion closest to a side fusing portion is detected by a detection unit.
FIG. 9 is an explanatory plan view of a method for manufacturing an underpants-type wearable article according to a modification of the present invention, wherein a mode is illustrated where rubber threads are cut in a direction inclined with respect to a direction that the rubber threads extend in a rubber thread cutting step.

### Description of Embodiments

Hereinafter, an embodiment of the present invention is described with reference to attached drawings. The following embodiment is an example which embodies the present invention, and is not intended to limit the technical scope of the present invention.

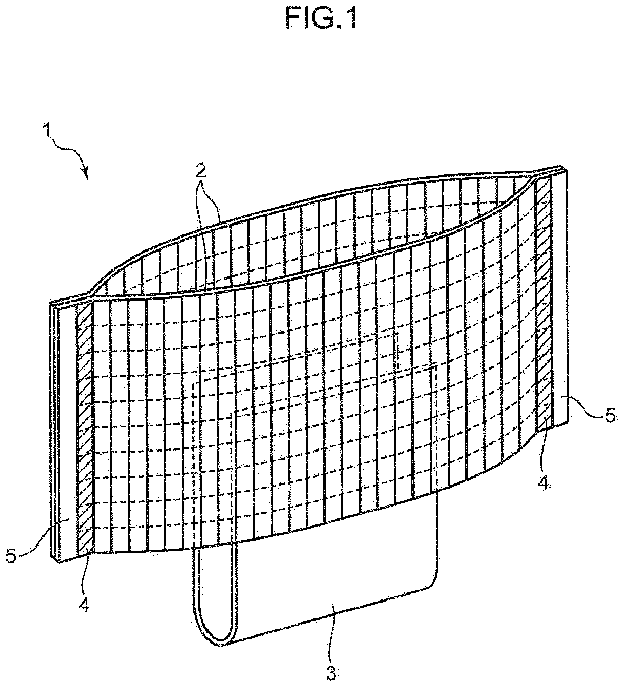

As illustrated in FIG. 1, an underpants-type wearable article 1 manufactured by the manufacturing method according to the embodiment of the present invention includes: a pair of waist members 2, that is, a front side waist member 2 disposed on a belly side of the body and a back side waist member 2 disposed on a back side of the body; and an absorbing body 3 disposed at a clotch portion of the body. The underpants-type wearable article 1 is manufactured by joining both side portions of the front side waist member and back side waist member 2 respectively. Both side portions are joined to each other by side fusing (side joining) using the pair of side fusing portions 4 (side joining portions). Protruding portions 5 that are not fused are disposed outside the pair of side fusing portions 4. In FIG. 1, to allow a viewer to easily visually recognize the protruding portion 5, the protruding portion 5 is depicted such that the protruding portion 5 has substantially the same width as the side fusing portion 4. However, in an actual product, a width of the protruding portion 5 may be set narrower than a width of the side fusing portion 4.

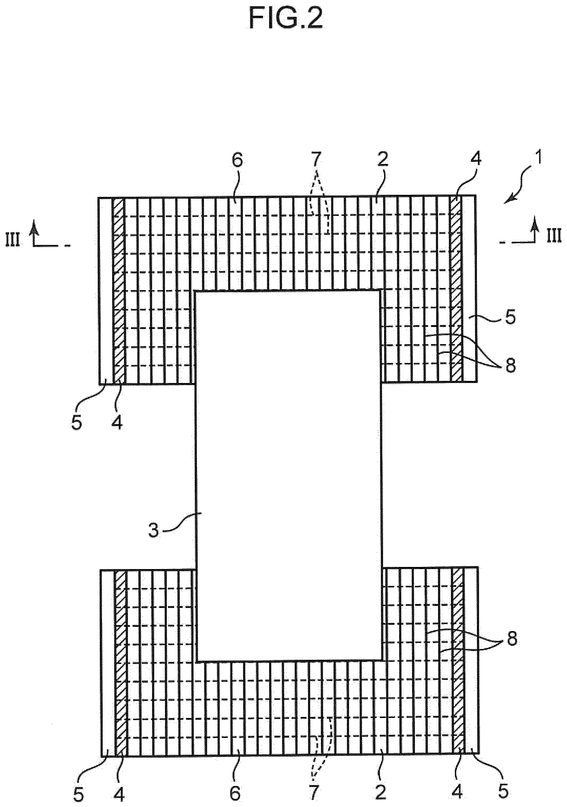

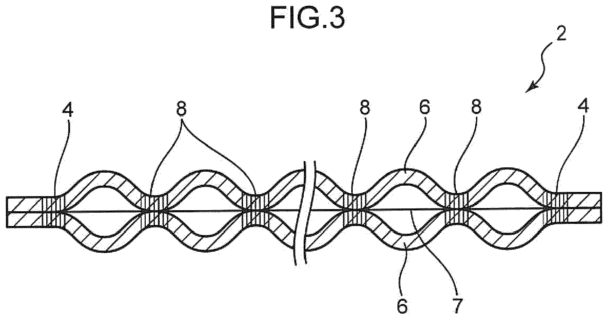

As illustrated in FIG. 2 and FIG. 3, each of the pair of waist members 2 includes: a pair of nonwoven fabrics 6 (sheets) made of a thermoplastic resin or the like; and a plurality of rubber threads 7 (elastic stretchable members) sandwiched between the pair of nonwoven fabrics 6. The sheet according to the present invention also includes, besides the nonwoven fabric 6, other sheet-shaped materials (for example, a woven fabric, a resin-made sheet and the like).

The plurality of rubber threads 7 are linear members having stretching and shrinking property, and are exemplified as one example of an elastic stretchable member the present invention. The rubber threads 7 are arranged in a spaced-apart manner in a width direction (a vertical direction in FIG. 2) of the nonwoven fabric 6. The plurality of rubber threads 7 are sandwiched between the pair of nonwoven fabrics 6 in a state where the plurality of rubber threads 7 extend in a predetermined stretching direction (a longitudinal direction of the nonwoven fabric 6, specifically, a left and right direction in FIG. 2). The plurality of rubber threads 7 are joined to each other by a plurality of joining portions 8.

The joining portions 8 linearly extend in a direction that intersects with the stretching direction (in the left and right directions in FIG. 2) of the rubber threads 7 (the direction orthogonal to the rubber threads 7 in the present embodiment). The joining portions 8 are formed by joining the pair of nonwoven fabric 6 and the rubber thread 7 by supersonic welding, heat sealing or the like in a state where the rubber threads 7 are sandwiched between the pair of nonwoven fabrics 6. The joining portions 8 join the pair of nonwoven fabrics 6 and the rubber threads 7 in a stretched state intermittently in a spaced-apart manner in the stretching direction (the left and right direction in FIG. 2).

In the present invention, it is sufficient that the joining portions 8 extend linearly in the direction orthogonal to the stretching direction of the rubber threads 7 (the left and right direction in FIG. 2), besides the direction orthogonal to the rubber threads 7 illustrated in FIG. 2, the joining portion 8 may extend in a direction inclined at an angle of 0 degree to less than 90 degree with respect to the stretching direction.

The absorbing body 3 is formed of a base material such as a nonwoven fabric, and an absorbing material which is dispersed in the base material. The absorbing material is made of a powdery or granular resin material capable of absorbing a liquid. Both end portions of the absorbing body 3 made to overlap with and are joined by adhesion or heat welding to the pair of front and back waist members 2, that is, the front side waist member 2 and the back side waist member 2 respectively.

The absorbing body 3 may be integrally formed with the pair of waist members 2.

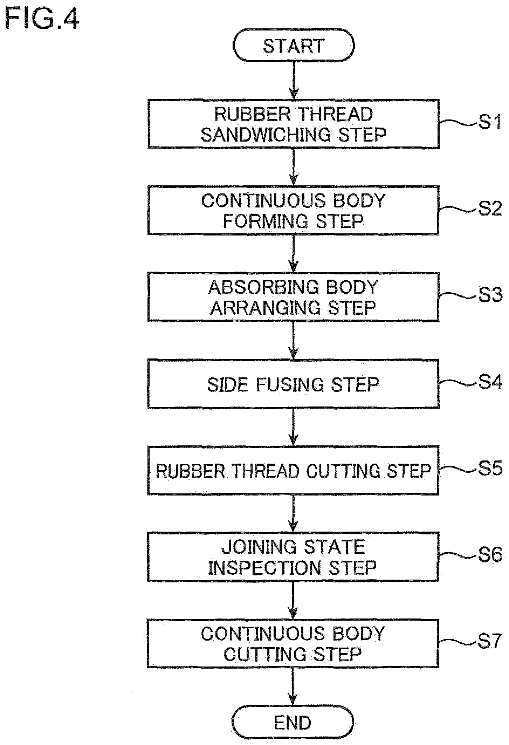

in a case where the underpants-type wearable article 1 having the above-mentioned configuration is manufactured by the manufacturing method of the present embodiment, the underpants-type wearable article 1 is manufactured in accordance with steps described in a flowchart illustrated in FIG. 4.

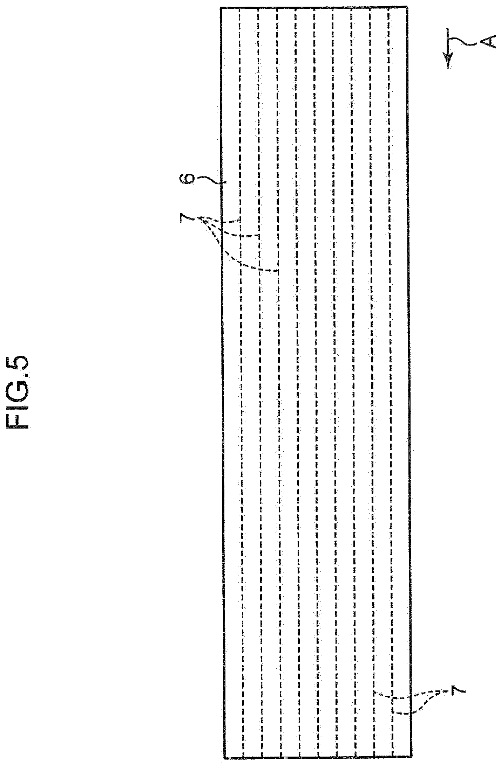

First, as illustrated in FIG. 5, a rubber thread sandwiching step (S1 in FIG. 4) is performed where the plurality of rubber threads 7 are sandwiched between the pair of nonwoven fabrics 6. Specifically, in the rubber thread sandwiching step, during the conveyance of the pair of elongated nonwoven fabrics 6 in a conveyance direction A, the plurality of rubber threads 7 extending in a predetermined stretching direction (the direction equal to the conveyance direction A) are sandwiched between the pair of nonwoven fabrics 6 in a direction orthogonal to the stretchable direction from each other at positions spaced-apart from each other.

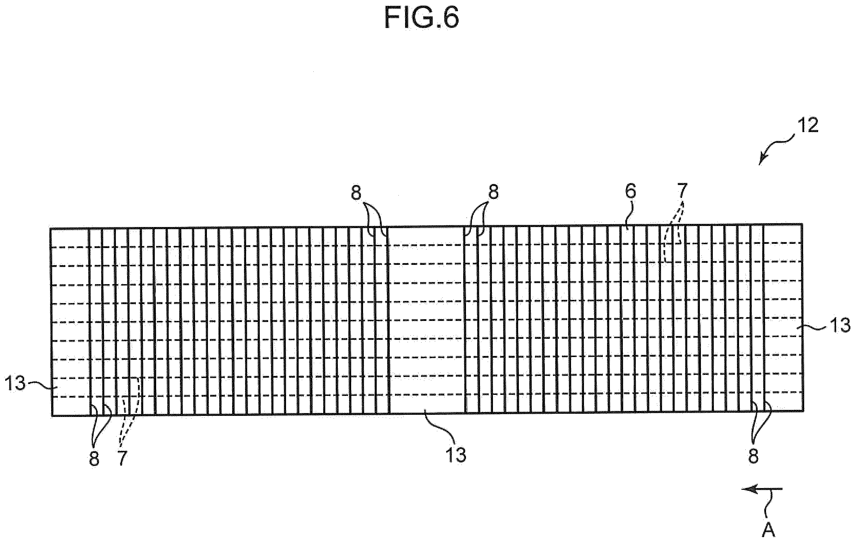

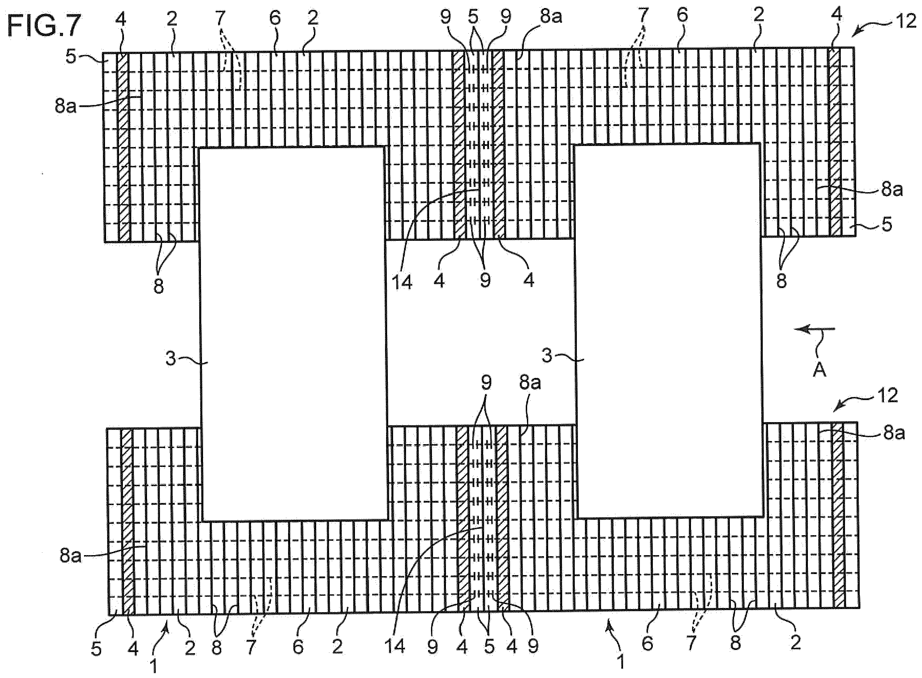

Then, as illustrated in FIG. 6, a step of forming the continuous body on a front side and the continuous body on a back side is performed (S2 in FIG. 4). In the step, the pair of nonwoven fabrics 6 and the plurality of rubber threads 7 in a stretched state are intermittently joined to each other at a fixed interval in the stretching direction (that is, the conveyance direction A) by the plurality of joining portions 8. Accordingly, a continuous body 12 of the front side waist members 2 and a continuous body 12 of the back side waist members 2 are formed respectively. Joining between the pair of nonwoven fabrics 6 and the plurality of rubber threads 7 may be performed by ultrasonic wave welding or heat sealing. In this continuous body forming step, the joining portions 8 are arranged such that the joining portions 8 are not formed on side forming portions 13 that form boundary regions on both sides of the waist member 2 (specifically, side fusing portions 4 and protruding portions 5 illustrated in FIG. 1, FIG. 2 and FIG. 7). That is, the joining portion 8 is formed only on portions of the waist member 2, the portions being regions sandwiched by the side fusing portions 4 (see FIG. 7).

Next, an absorbing body arranging step is performed (S3 in FIG. 4). That is, as illustrated in FIG. 7, the absorbing bodies 3 are arranged such that both end portions of each absorbing body 3 overlap with the continuous body 12 of the front side waist members 2 and the continuous body 12 of the back side waist members 2 respectively in a state where the continuous body 12 of the front side waist members 2 and the continuous body 12 of the back side waist members 2 are conveyed in the conveyance direction in parallel to each other. The absorbing bodies 3 are sequentially arranged at an interval in the conveyance direction A above the continuous body 12 of the front side waist members 2 and the continuous body 12 of the back side waist members 2.

In a case where the absorbing body 3 is integrally formed with the pair of waist members 2, the absorbing body arranging step S3 may be omitted.

Next, a side fusing step is performed (S4 in FIG. 4). As illustrated in FIG. 7, the continuous body 12 of the front side waist members 2 and the continuous body 12 of the back side waist members 2 are made to overlap with each other, and the front side waist members 2 and the back side waist members 2 are intermittently fused to each other at the side fusing portion 4 in a boundary region which is a boundary of an individual wearable article. The side fusing portion 4 may perform side fusing by ultrasonic fusing or heat sealing.

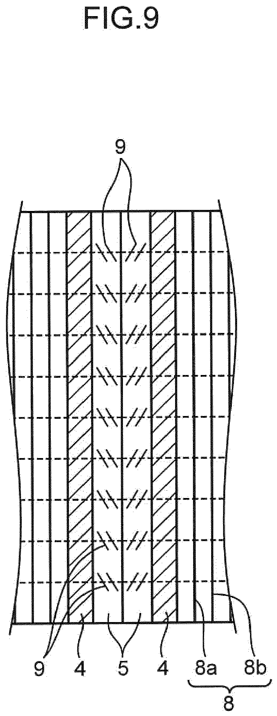

After the side fusing step is finished, a rubber thread cutting step is performed (S5 in FIG. 4). As illustrated in FIG. 7, among boundary regions which form boundaries of the individual wearable article 1 between the pair of side fusing portions 4 and another pair of side fusing portions 4 disposed adjacently to the pair of side fusing portions 4, in the protruding portions 5 of each wearable article 1, the rubber threads 7 in the continuous body 12 of the front side waist members 2, and the rubber thread 7 in the continuous body 12 of the back side waist members 2 are cut at cutting portions 9. In cutting the rubber threads 7, in each protruding portion 5 sandwiched between the side fusing portions 4 disposed adjacently to each other, only the rubber threads 7 are cut without cutting the nonwoven fabric 6 by a cutting unit such as a dot cutter (that is, a cutter where a large number of small blades are mounted on a peripheral surface of a cylindrical roll). In the rubber thread cutting step, as in the case of a cutting portion 9 illustrated in FIG. 9, the rubber threads 7 may be cut in a direction inclined with respect to a direction that the rubber threads 7 extend.

Next, a joining state inspection step is performed (S6 in FIG. 4). In this step, a joining state of the rubber threads 7 to the pair of nonwoven fabrics 6, that is, a joining state of the joining portions 8 is inspected.

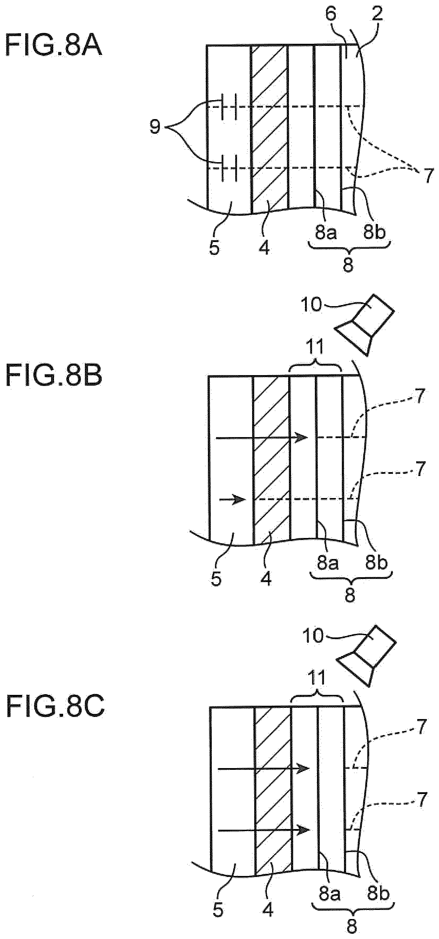

Specifically, in the joining state inspection step, as illustrated in FIGS. 8A to 8 C, among a plurality of joining portions 8 in each wearable article 1, a joining defect at a joining portion 8a between the nonwoven fabric 6 closest to the side fusing portion 4 and the rubber threads 7 in the boundary region of each wearable article 1 is detected.

First, as illustrated in FIG. 8A, when the rubber threads 7 are cut by the cutting portion 9 in the protruding portion 5, rubber threads 7 are shrunken. As illustrated in FIG. 8B, in a case where a joining state of the rubber threads 7 at the joining portion 8a closest to the side fusing portion 4 is favorable, end portions of the rubber threads 7 stop at the joining portion 8a. In this case, the rubber threads 7 are detected in the vicinity of the joining portion 8a by monitoring a monitoring region 11 in the vicinity of the joining portion 8a (for example, a region between the side fusing portion 4 and the joining portion 8b disposed adjacently to the joining portion 8a) using a detection unit 10 such as a television camera or an optical sensor. With such an operation, it is possible to detect that a joining state at the joining portion 8a is favorable. Also in a case where the rubber threads 7 are stopped by being fused to a portion of the side fusing portion 4 as in the case of the rubber threads 7 on a lower side in FIG. 8B, the rubber threads 7 are detected in the monitoring region 11 and hence, it is possible to detect that the joining state is favorable.

On the other hand, as illustrated in FIG. 8C, in a case where a joining state of the rubber threads 7 at the joining portion 8a closest to the side fusing portion 4 is defective, end portions of the rubber threads 7 do not stop at the joining portion 8a, and is shrunken to the joining portion 8b disposed adjacently to the joining portion 8a or a portion away from the joining portion 8b. At this stage of operation, the detection unit 10 detects that no rubber threads 7 exist in the monitoring region 11 in the vicinity of the joining portion 8a. Accordingly, it is possible to detect that a joining state at the joining portion 8a is defective.

When it is determined that the joining state of the rubber threads 7 is defective in the inspection step, it is preferable to include an abnormality notification step which notifies an operator of the occurrence of abnormality.

In a case where the joining state is defective, an operator stops the manufacture of the wearable article, removes defective parts of the waist member 2, and performs inspection, adjustment and the like of the manufacturing apparatus.

Then, a continuous body cutting step is performed (S7 in FIG. 4). In a case where it is detected that a joining state of the rubber threads 7 is favorable in the joining state inspection step, as illustrated in FIG. 7, the continuous body 12 of the front side waist members 2 and the continuous body 12 of the back side waist members 2 are cut at the cutting portion 14 between two protruding portions 5 disposed adjacently to each other among the boundary regions of each wearable article 1. Accordingly, the individual wearable articles 1 can be obtained. In accordance with the above-mentioned steps, the underpants-type wearable articles 1 can be continuously manufactured.

The rubber thread cutting step S5 may be performed before the side fusing step S4 where the front side waist members 2 and the back side waist members 2 are fused at the side fusing portion 4. In that case, in the rubber thread cutting step S5, the rubber threads 7 in the continuous body 12 of the front side waist members 2, and the rubber threads 7 in the continuous body 12 of the back side waist members 2 may be cut by the cutting portion 9 in the side forming portions 13 illustrated in FIG. 6 which become the boundary regions of the individual wearable articles 1 (specifically, portions which are scheduled to become the protruding portions among the portions in a state before the side fusing portion 4 and the protruding portions 5 are formed).

### (Characteristics of the present embodiment)

(1) As described above, the method for manufacturing the underpants-type wearable article 1 of the present embodiment includes: the rubber thread sandwiching step S1; the front side and back side continuous body forming step S2 of forming the continuous body 12 of the front side waist member 2 and the continuous body 12 of the back side waist member 2 respectively; the side fusing step S4 of intermittently fusing the front side waist member 2 and the back side waist member 2 by the side fusing portion 4; the rubber thread cutting step S5 of cutting the rubber threads 7 in the continuous body 12 of the front side waist member 2 and the continuous body 12 of the back side waist member 2 at the boundary that forms the boundary region of the individual wearable article 1 (specifically, the protruding portion 5); the joining state inspection step S6 of inspecting a joining state of the rubber thread 7 to the nonwoven fabric 6; and continuous body cutting step S7 of obtaining the individual wearable article 1 by cutting the continuous body 12 of the front side waist member 2 and the continuous body 12 of the back side waist member 2 at the boundary region (specifically, the protruding portion 5).

In this manufacturing method, in a state before the individual wearable articles are obtained by cutting the continuous body 12 of the front side waist member 2 and the continuous body 12 of the back side waist member 2, that is, in a state where the rubber threads 7 are stretched before being shrunken by the separation of the continuous bodies 12 into the individual wearable articles, in the boundary regions which are the regions of the individual wearable articles in the continuous body 12 of the front side waist member 2 and the continuous body 12 of the back side waist member 2, the rubber threads 7 in the continuous body 12 of the front side waist member 2 and the continuous body 12 of the back side waist member 2 are respectively cut. As a result, in a state where the joining of the rubber threads 7 is defective, the end portions of the rubber threads 7 move due to a surface tension of the rubber threads 7 in a state where the respective nonwoven fabrics 6 (sheets) in the continuous body 12 of the front side waist member 2 and the continuous body 12 of the back side waist member 2. Accordingly, a joining state of the rubber thread 7 to the pair of nonwoven fabric 6 can be easily checked.

(2)
In the method for manufacturing the underpants-type wearable article 1 of the present embodiment, the side fusing step S4 is performed before the rubber thread cutting step S5. In this case, even when a joining defect occurs between the rubber threads 7 and the pair of nonwoven fabrics 6 in the continuous body forming step S2, in the side fusing step S4, when the individual front side waist member 2 and the individual back side waist member 2 are fused by side-fusing at the side fusing portion 4, the rubber threads 7 may be connected to the nonwoven fabrics 6 at the side fusing portion 4 and hence, a joining defect is eliminated. Accordingly, a joining defect of the rubber threads 7 can be reduced.

(3)
In the method for manufacturing the underpants-type wearable article 1 of the present embodiment, in the joining state inspection step S6, a joining defect at the joining portion between the nonwoven fabric 6 and the rubber threads 7 closest to the boundary region (the protruding portion 5 or a part of the protruding portion 5 in the side forming portion 13 that becomes the protruding portion 5) is detected.

Provided that the favorable joining portion is ensured between the nonwoven fabric 6 and the rubber threads 7 closest to the boundary region, even when a joining defect occurs at the joining portion away from the boundary region, stretching and shrinking of the rubber threads 7 can be prevented by the joining portion closest to the boundary region. In other words, it is possible to detect a joining state of the rubber threads 7 of each wearable article over the entire length of the rubber threads 7 only by detecting a joining state of the nonwoven fabric 6 and the rubber threads 7 closest to the boundary region. In view of the above, in the manufacturing method described above, in the joining state inspection step S6, by detecting a joining defect at the joining portion between the nonwoven fabric 6 and the rubber threads 7 closest to the boundary region, a joining defect of the rubber threads 7 can be easily detected with certainty before the continuous body cutting step S7.

(4)
In the method for manufacturing the underpants-type wearable article 1 of the present embodiment, it is preferable that the method further includes an abnormality notification step of notifying the occurrence of abnormality when a joining state of the rubber thread 7 is determined to be defective in the joining state inspection step S6. With such a configuration, an operator knows the occurrence of abnormality of a joining defect of the rubber thread 7.

### (Modification)

(A) In the manufacturing method of the present embodiment, the front side waist member 2 and the back side waist member 2 are joined to each other by side fusing (side joining). However, the present invention is not limited to such joining, and side joining may be performed by joining other joining besides fusing, for example, by adhesion.
(B) Further, in the manufacturing method of the present embodiment, the front side waist member 2 and the back side waist member 2 are formed in a separated form. However, the present invention is not limited to such a configuration.

As a modification of the present invention, the front side waist member 2 and the back side waist member 2 may be formed in a form where the front side waist member 2 and the back side waist member 2 are connected to each other (that is, in a form where the front side waist member 2 and the back side waist member 2 are connected to and are integrally formed with an inseam portion). In this case, in the manufacturing method of the present invention, the front side continuous body forming step and the back side continuous body forming step are performed simultaneously.

### <Recapitulation of embodiment>

The above embodiment is recapitulated as follows.

The method for manufacturing an underpants-type wearable article according to the present embodiment is a method for manufacturing an underpants-type wearable article that includes: a front side waist member disposed on a belly side of a body; a back side waist member disposed on a back side of the body; and an absorbing body disposed at a clotch portion of the body, both side portions of the front side waist member and the back side waist member being joined to each other by side joining using a pair of side joining portions, the method including: an elastic stretchable member sandwiching step of sandwiching a plurality of elastic stretchable members stretched in a predetermined stretching direction between a pair of sheets; a front side continuous body forming step, performed after the elastic stretchable member sandwiching step, of forming a continuous body of the front side waist members by intermittently joining, in the stretching direction, the pair of sheets and the elastic stretchable members in a stretched state; a back side continuous body forming step, performed after the elastic stretchable member sandwiching step, of forming a continuous body of the back side waist members by intermittently joining, in the stretching direction, the pair of sheets and the elastic stretchable member in a stretched state; a side joining step of intermittently joining the front side waist member and the back side waist member by the side joining portions in boundary regions which become boundaries of individual wearable articles in a state where the continuous body of the front side waist members and the continuous body of the back side waist members are overlapped with each other; an elastic stretchable member cutting step of cutting the elastic stretchable member of a continuous body of the front side waist members and the elastic stretchable members of the continuous body of the back side waist member in the boundary regions in the continuous body of the front side waist members and the continuous body of the back side waist members without cutting the sheets; a joining state inspection step, performed after the elastic stretchable member cutting step, of inspecting a joining state of the elastic stretchable members to the pair of sheet; and a continuous body cutting step, performed after the joining state inspection step, of obtaining individual wearable articles by cutting the continuous body of the front side waist members and the continuous body of the back side waist members at the boundary regions.

In such a manufacturing method, the continuous body of the front side waist members and the continuous body of the back side waist members are cut at boundary regions which become boundaries of the individual wearable articles in a state before the continuous body of the front side waist members and the continuous body of the back side waist members are cut so as to obtain the individual wearable article, that is, in a state where the elastic stretchable members are stretched before the elastic stretchable member are shrunken. As a result, when the joining of the elastic stretchable member is defective, the end portion of the elastic stretchable member moves due to the tension of the elastic stretchable member in a state where the sheet is stretched. Accordingly, a joining state of the elastic stretchable member to the pair of sheets ca be easily checked.

In the above manufacturing method, it is preferable that the side joining step is performed before the elastic stretchable member cutting step.

In such a manufacturing method, by performing the side joining step before the elastic stretchable member cutting step, even when a joining defect occurs between the elastic stretchable member and the pair of sheets during the continuous body forming step, in the side joining step, at the time of joining the individual front side waist member and the back side waist member by the side joining portion, the elastic stretchable member may be connected to the sheet by the side joining portion and hence, a joining defect is eliminated. Accordingly, the joining defect of the elastic stretchable member can be reduced.

In the above manufacturing method, it is preferable that in the inspection step, a joining defect in the joining portion between the sheet and the elastic stretchable member closest to the boundary region is detected.

Provided that the favorable joining portion is ensured between the sheet closest to the boundary region and the elastic stretchable member, even when a joining defect occurs at the joining portion away from the boundary region, the joining portion closest to the boundary region can prevent the elastic stretchable member from being stretched or shrunken. In other words, it is possible to detect the joining state of the elastic stretchable member of each wearable article over the entire length only by detecting the joining state between the sheet closest to the boundary region and the elastic stretchable member. Therefore, in the above manufacturing method, in the inspection step, by detecting a joining defect at the joining portion between the sheet closest to the boundary region and the elastic stretchable member, the joining defect of the elastic stretchable member can be detected easily with certainty before the continuous body cutting step.

In the above manufacturing method, it is preferable that the manufacturing method further includes an abnormality notification step of notifying an occurrence of abnormality when the joining state of the elastic stretchable member is determined to be defective in the inspection step. With such a configuration, an operator knows the occurrence of abnormality of a joining defect with certainty.

## Claims

1. A method for manufacturing an underpants-type wearable article (1) that includes: a front side waist member (2) disposed on a belly side of a body; a back side waist member (2) disposed on a back side of the body; and an absorbing body (3) disposed at a clotch portion of the body, both side portions of the front side waist member (2) and the back side waist member (2) being joined to each other by side joining using a pair of side joining portions (4), the method comprising:
an elastic stretchable member sandwiching step of sandwiching a plurality of elastic stretchable members (7) stretched in a predetermined stretching direction between a pair of sheets (6);
a front side continuous body forming step, (S2) performed after the elastic stretchable member sandwiching step, of forming a continuous body (12) of the front side waist members (2) by intermittently joining, in the stretching direction, the pair of sheets (6) and the elastic stretchable members (7) in a stretched state;
a back side continuous body forming step, (S2) performed after the elastic stretchable member sandwiching step, of forming a continuous body (12) of the back side waist members (2) by intermittently joining, in the stretching direction, the pair of sheets (6) and the elastic stretchable members (7) in a stretched state;
a side joining step of intermittently joining (S4) the front side waist members (2) and the back side waist members (2) by the side joining portions (4) in boundary regions which become boundaries of individual wearable articles (1) in a state where the continuous body (12) of the front side waist members (2) and the continuous body (12) of the back side waist members (2) are overlapped with each other;
an elastic stretchable member cutting step (S5) of cutting the elastic stretchable members (7) of the continuous body (12) of the front side waist members (12) and the elastic stretchable members (7) of the continuous body (12) of the back side waist member (12) in the individual boundary regions in the continuous body (12) of the front side waist members (2) and the continuous body (12) of the back side waist members (2) without cutting the sheets (6);
a joining state inspection step, (S6) performed after the elastic stretchable member cutting step, of inspecting a joining state of the elastic stretchable members (7) to the pair of sheets (6); and
a continuous body cutting step, (S7) performed after the joining state inspection step of obtaining individual wearable articles (1) by cutting the continuous body (2) of the front side waist members (12) and the continuous body (2) of the back side waist members (12) at the boundary regions.

2. The method for manufacturing an underpants-type wearable article (1) according to claim 1, wherein the side joining step is performed before the elastic stretchable member cutting step.

3. The method for manufacturing an underpants-type wearable article (1) according to claim 1 or 2, wherein, in the inspection step, a joining defect in the joining portion between the sheet (6) and the elastic stretchable member (7) closest to the boundary region is detected.

4. The method for manufacturing an underpants-type wearable article (1) according to any one of claims 1 to 3, further comprising
An abnormality notification step of notifying an occurrence of abnormality when the joining state of the elastic stretchable member (7) is determined to be defective in the inspection step.

## Patentansprüche

1. Verfahren zur Herstellung eines unterhosenartigen, tragbaren Artikels (1), der Folgendes umfasst: ein vorderseitiges Taillenelement (2), das an einer Bauchseite eines Körpers angeordnet ist; ein rückseitiges Taillenelement (2), das an einer Rückseite des Körpers angeordnet ist; und einen absorbierenden Körper (3), der an einem Klammerabschnitt des Körpers angeordnet ist, wobei beide Seitenabschnitte des vorderseitigen Taillenelements (2) und des rückseitigen Taillenelements (2) durch seitliches Verbinden unter Verwendung eines Paars von Seitenverbindungsabschnitten (4) miteinander verbunden werden, wobei das Verfahren Folgendes umfasst:
einen Sandwich-Schritt für elastische dehnbare Elemente, bei dem eine Vielzahl von elastischen dehnbaren Elementen (7), die in einer vorbestimmten Dehnungsrichtung gedehnt werden, zwischen einem Paar von Lagen (6) sandwichartig angeordnet werden;
einen Schritt (S2) zum Bilden eines kontinuierlichen Körpers auf der Vorderseite, der nach dem Schritt des Einlegens der elastischen dehnbaren Elemente durchgeführt wird, zum Bilden eines kontinuierlichen Körpers (12) der Taillenelemente (2) auf der Vorderseite durch intermittierendes Verbinden des Paares von Lagen (6) und der elastischen dehnbaren Elemente (7) in der Dehnungsrichtung in einem gedehnten Zustand;
einen Schritt (S2) zum Bilden eines kontinuierlichen Rückseitenkörpers, der nach dem Schritt des Einfügens des elastischen dehnbaren Elements durchgeführt wird, zum Bilden eines kontinuierlichen Körpers (12) der Rückseiten-Taillenelemente (2) durch intermittierendes Verbinden des Paars von Lagen (6) und der elastischen dehnbaren Elemente (7) in der Dehnungsrichtung in einem gedehnten Zustand;
einen Seitenverbindungsschritt des intermittierenden Verbindens (S4) der Vorderseiten-Taillenelemente (2) und der Rückseiten-Taillenelemente (2) durch die Seitenverbindungsabschnitte (4) in Grenzbereichen, die in einem Zustand, in dem der durchgehende Körper (12) der Vorderseiten-Taillenelemente (2) und der durchgehende Körper (12) der Rückseiten-Taillenelemente (2) einander überlappen, zu Grenzen von einzelnen tragbaren Artikeln (1) werden;
einen Schritt (S5) des Schneidens der elastischen dehnbaren Elemente (7) des durchgehenden Körpers (12) der Vorderseiten-Taillenelemente (12) und der elastischen dehnbaren Elemente (7) des durchgehenden Körpers (12) des Rückseiten-Taillenelements (12) in den einzelnen Grenzbereichen in dem durchgehenden Körper (12) der Vorderseiten-Taillenelemente (2) und dem durchgehenden Körper (12) der Rückseiten-Taillenelemente (2) ohne Schneiden der Lagen (6);
einen Schritt (S6) zur Überprüfung des Verbindungszustands, der nach dem Schritt des Schneidens der elastischen dehnbaren Elemente durchgeführt wird, um den Verbindungszustand der elastischen dehnbaren Elemente (7) mit dem Paar von Lagen (6) zu überprüfen; und
einen Schritt (S7) des Schneidens eines kontinuierlichen Körpers, der nach dem Schritt der Prüfung des Verbindungszustands durchgeführt wird, um einzelne tragbare Artikel (1) durch Schneiden des kontinuierlichen Körpers (2) der vorderen Taillenelemente (12) und des kontinuierlichen Körpers (2) der hinteren Taillenelemente (12) an den Grenzbereichen zu erhalten.

2. Verfahren zur Herstellung eines unterhosenartigen tragbaren Artikels (1) nach Anspruch 1, wobei der Schritt des seitlichen Verbindens vor dem Schritt des Schneidens des elastischen dehnbaren Elements durchgeführt wird.

3. Verfahren zur Herstellung eines unterhosenartigen, tragbaren Artikels (1) nach Anspruch 1 oder 2, wobei in dem Inspektionsschritt ein Verbindungsfehler in dem Verbindungsabschnitt zwischen der Folie (6) und dem elastischen, dehnbaren Element (7), der dem Grenzbereich am nächsten liegt, erkannt wird.

4. Verfahren zur Herstellung eines unterhosenartigen, tragbaren Artikels (1) nach einem der Ansprüche 1 bis 3, ferner umfassend
einen Anomalienmeldeschritt, in dem das Auftreten einer Anomalie gemeldet wird, wenn der Verbindungszustand des elastischen dehnbaren Elements (7) in dem Inspektionsschritt als fehlerhaft festgestellt wird.

## Revendications

1. Procédé de fabrication d'un article portable de type caleçon (1) comprenant : un élément de taille avant (2) disposé sur le côté ventral d'un corps ; un élément de taille arrière (2) disposé sur le côté arrière du corps ; et un corps absorbant (3) disposé au niveau d'une partie d'encoche du corps, les deux parties latérales de l'élément de taille avant (2) et de l'élément de taille arrière (2) étant reliées l'une à l'autre par assemblage latéral à l'aide d'une paire de parties d'assemblage latéral (4), le procédé comprenant :
une étape de prise en sandwich d'éléments élastiques étirables consistant à prendre en sandwich une pluralité d'éléments élastiques étirables (7) étirés dans une direction d'étirement prédéterminée entre une paire de feuilles (6) ;
une étape de formation d'un corps continu sur la face avant (S2), réalisée après l'étape de prise en sandwich des éléments élastiques étirables, consistant à former un corps continu (12) des éléments de taille de la face avant (2) en assemblant par intermittence, dans la direction d'étirement, la paire de feuilles (6) et les éléments élastiques étirables (7) à l'état étiré ;
une étape de formation d'un corps continu sur la face arrière, (S2) réalisée après l'étape de prise en sandwich des éléments élastiques étirables, consistant à former un corps continu (12) des éléments de taille de la face arrière (2) en assemblant par intermittence, dans la direction d'étirement, la paire de feuilles (6) et les éléments élastiques étirables (7) à l'état étiré ;
une étape d'assemblage latéral consistant à assembler par intermittence (S4) les éléments de taille du côté avant (2) et les éléments de taille du côté arrière (2) par les parties d'assemblage latéral (4) dans les régions limitrophes qui deviennent les limites des articles portables individuels (1) dans un état où le corps continu (12) des éléments de taille du côté avant (2) et le corps continu (12) des éléments de taille du côté arrière (2) sont superposés l'un sur l'autre ;
une étape de découpe des éléments élastiques étirables (S5) consistant à découper les éléments élastiques étirables (7) du corps continu (12) des membres de la taille du côté avant (12) et les éléments élastiques étirables (7) du corps continu (12) du membre de la taille du côté arrière (12) dans les régions limites individuelles du corps continu (12) des membres de la taille du côté avant (2) et du corps continu (12) des membres de la taille du côté arrière (2), sans découper les feuilles (6) ;
une étape d'inspection de l'état d'assemblage, (S6) réalisée après l'étape de découpe des éléments élastiques étirables, consistant à inspecter l'état d'assemblage des éléments élastiques étirables (7) à la paire de feuilles (6) ; et
une étape de découpe du corps continu (S7), réalisée après l'étape d'inspection de l'état d'assemblage, consistant à obtenir des articles portables individuels (1) en découpant le corps continu (2) des éléments de taille du côté avant (12) et le corps continu (2) des éléments de taille du côté arrière (12) au niveau des zones de délimitation.

2. Procédé de fabrication d'un article vestimentaire de type sous-pantalon (1) selon la revendication 1, dans lequel l'étape d'assemblage latéral est réalisée avant l'étape de découpe de l'élément élastique extensible.

3. Procédé de fabrication d'un article portable de type sous-pantalon (1) selon la revendication 1 ou 2, dans lequel, lors de l'étape d'inspection, un défaut d'assemblage est détecté dans la partie d'assemblage entre la feuille (6) et l'élément élastique extensible (7) la plus proche de la zone de délimitation.

4. Le procédé de fabrication d'un article portable de type sous-pantalon (1) selon l'une quelconque des revendications 1 à 3, comprenant en outre
Une étape de notification d'anomalie consistant à notifier l'apparition d'une anomalie lorsque l'état d'assemblage de l'élément élastique extensible (7) est jugé défectueux lors de l'étape d'inspection.
